# EUROPEAN PATENT APPLICATION

(11) **EP 4 556 113 A1**
(43) Date of publication of application: **21.05.2025**
(21) Application number: 24191932.3
(22) Date of filing: 31.07.2024
(51) Int. Cl.: B01J 23/04, B01J 29/40, C07C 69/54

(54) **METHOD OF PRODUCING CATALYST FOR SYNTHESIZING ALKYL ACRYLATE FROM ALKYL LACTATE AND A METHOD OF PRODUCING ALKYL ACRYLATE USING CATALYST**

(30) Priority: 16.11.2023 KR 20230158978
(71) Applicant: Hyundai Motor Company, Seoul 06797 (KR); Kia Corporation, Seocho-gu Seoul 06797 (KR); Korea Research Institute of Chemical Technology, Daejeon 34114 (KR)
(72) Inventor: SUNG, Jong Geun, 18280 Hwaseong-si, Gyeonggi-do (KR); KIM, Da Bin, 18280 Hwaseong-si, Gyeonggi-do (KR); JEON, Sung Wan, 18280 Hwaseong-si, Gyeonggi-do (KR); HWANG, Dong Won, 34114 Daejeon (KR); CHA, Seung Hyeok, 34114 Daejeon (KR); RYU, Tae Kyung, 34114 Daejeon (KR)
(74) Representative: Isarpatent

(57) **Abstract**

A method of producing a catalyst for synthesizing alkyl acrylate from alkyl lactate and a method of producing alkyl acrylate using the catalyst are disclosed herein. More specifically, a catalyst including zeolite is produced by a method that does not use an organic structure directing agent, and a dehydration reaction of alkyl lactate is promoted using the catalyst, thereby obtaining alkyl acrylate in a yield higher than obtained by conventional methods.

## Description

### BACKGROUND

### Field

The present disclosure relates to a method of producing a catalyst for synthesizing alkyl acrylate from alkyl lactate and a method of producing alkyl acrylate using the catalyst.

### Description of the Related Art

Acrylic acid (AA) is a type of alpha-beta unsaturated carboxylic acid that possesses both a double bond and a functional group, and is used in various chemical reactions due to its excellent reactivity. Acrylic acid may be polymerized with itself (addition polymerization) or with other monomers (copolymerization) to create various polymers. Resin obtained through polymerization of acrylic acid dissolves in water to form a highly viscous solution, so it may be used to increase viscosity in lacquers, varnishes, inks, and the like.

In addition, when acrylic acid is hydrolyzed by adding alcohol, an ester in the form of alkyl acrylate can be formed. Alkyl acrylates, such as methyl acrylate (MA), ethyl acrylate (EA), butyl acrylate (BA), and 2-ethylhexyl acrylate (EHA), are very useful substances used in various fields such as adhesives, paints, inks, textile treatments, resins, antioxidants, and construction materials.

Acrylates can be synthesized by adding alcohol to acrylic acid. Methods for synthesizing acrylic acid include two oxidation reactions of allyl alcohol, dehydrochlorination reaction of chloropropionic acid, carbonylation reaction of acetylene, or dehydration reaction of lactic acid.

Currently, most acrylates are produced by vapor-phase oxidation method of propylene, but recently, a method of producing acrylic acid or acrylate by dehydrating lactic acid or lactate has been attracting attention. This is because it is a method that uses biomass-derived materials rather than fossil fuels.

In 2019, the U.S. National Renewable Energy Laboratory (NREL) disclosed a method of obtaining lactic acid or methyl lactate using raw materials including lignocellulose and then synthesizing methyl acrylate and acrylonitrile (Acrylate production from lignocellulosic feedstocks, Violeta Sanchezi Nogue, 40th Symposium on Biotechnology for Fuels and Chemicals, Non-Patent Document 1). However, because the synthesis method disclosed in Non-Patent Document 1 requires an intermediate product such as methyl 2-acetoxy propionate, the synthesis time may be long and the yield may be low.

As a related art to solve this problem, Korean Patent Application Publication No. 2020-0079316 (published on July 2, 2020, Patent Document 1) discloses a method of producing methyl acrylate by contacting methyl lactate with a ZSM-5 catalyst in the presence of methanol. Patent Document 1 is characterized by using lactate rather than lactic acid as a reactant, and methyl acrylate can be formed directly by directly dehydrating methyl lactate without forming an intermediate product. In Patent Document 1, the ZSM-5 catalyst is a type of zeolite catalyst, and a commercial ZSM-5 catalyst is used. However, there is still room for improvement in the yield of the reaction for producing alkyl acrylate from alkyl lactate.

The foregoing is intended merely to aid in the understanding of the background of the present disclosure, and is not intended to mean that the present disclosure falls within the purview of the related art that is already known to those having ordinary skill in the art.

### SUMMARY

The present disclosure has been made keeping in mind the above problems occurring in the related art, and an objective of the present disclosure is to provide a catalyst capable of achieving lower cost and higher yield than obtained by conventional processes in the process of converting alkyl lactate to alkyl acrylate, and a process of producing alkyl acrylate using the catalyst.

The objectives of the present disclosure are not limited to those mentioned above. The objectives of the present disclosure become apparent from the following description and may be realized by means and combinations thereof as set forth in the claims.

In order to achieve the above objective, according to one aspect of the present disclosure, a method of producing alkyl acrylate is provided. The method includes: synthesizing a catalyst including a zeolite without using an organic structure directing agent; and obtaining alkyl acrylate by contacting alkyl lactate with the catalyst in presence of alkanol.

The synthesizing of the catalyst may include: preparing a mixed solution including an aluminum source, a silicon source, an alkali source and water; obtaining the zeolite by hydrothermal synthesis of the mixed solution; calcining the zeolite; and obtaining the catalyst by adding and reacting the zeolite to a solution including a potassium salt to ion-exchange the zeolite with potassium ions.

In the preparing of the mixed solution, the mixed solution may not include the organic structure directing agent.

The zeolite may include ZSM-5.

The zeolite may be represented by Chemical Formula 1:

xAl₂O₃·yNa₂O·100SiO₂·2500H₂O [Chemical Formula 1]

wherein x may be a number in a range of 1 to 7, and y may be a number in a range of 5 to 15.

The potassium salt may include potassium bromide (KBr).

The catalyst may have an Si/Al molar ratio in a range of 5:1 to 50:1.

The catalyst may have a K/Al molar ratio in a range of 0.6:1 to 1:1.

The alkanol and the alkyl lactate may include the same alkyl group.

The obtaining of the alkyl acrylate may be performed at a temperature in a range of 300°C to 400°C.

The obtaining of the alkyl acrylate may be performed by reacting 20% by weight to 90% by weight of the alkanol and 10% by weight to 80% by weight of the alkyl lactate with the catalyst to obtain the alkyl acrylate.

According to another aspect of the present disclosure, a method is provided for producing a catalyst that promotes a reaction for producing alkyl acrylate from alkyl lactate. The method includes: preparing a mixed solution including an aluminum source, a silicon source, an alkali source, and water; obtaining a zeolite by hydrothermal synthesis of the mixed solution; calcining the zeolite; and obtaining the catalyst by adding and reacting the zeolite to a solution including a potassium salt to ion-exchange the zeolite with potassium ions.

In the preparing the mixed solution, the mixed solution may not include an organic structure directing agent.

By using the catalyst according to the present disclosure, alkyl acrylate can be obtained in high yield when producing alkyl acrylate from alkyl lactate.

Because the organic structure directing agent is not used in the production of the catalyst, alkyl acrylate can be synthesized in an environmentally friendly manner.

The effects of the present disclosure are not limited to those mentioned above. The effects of the present disclosure should be understood to include all effects that can be inferred from the following description.

### BRIEF DESCRIPTION OF THE DRAWINGS

The above and other objectives, features, and other advantages of the present disclosure are more clearly understood from the following detailed description when taken in conjunction with the accompanying drawings, in which:
FIG. 1 shows the conversion rate of methyl lactate, selectivity of methyl acrylate, and yield of methyl acrylate in Experimental Example 1;
FIG. 2 shows the conversion rate of methyl lactate, selectivity of methyl acrylate, and yield of methyl acrylate in Experimental Example 2;
FIG. 3 shows the conversion rate of methyl lactate, selectivity of methyl acrylate, and yield of methyl acrylate in Experimental Example 3; and
FIG. 4 shows the conversion rate of methyl lactate, selectivity of methyl acrylate, and yield of methyl acrylate in Experiment Example 4.

### DETAILED DESCRIPTION

The above and other objectives, features, and advantages of the present disclosure are clearly understood from the more particular description of exemplary embodiments of the present disclosure. The present disclosure may, however, be embodied in different forms and should not be construed as limited to the embodiments set forth herein. Rather, these embodiments are provided so that this disclosure is thorough and complete and fully conveys the present disclosure to those skilled in the art.

Throughout the drawings, the same reference numerals refer to the same or like parts. Also, in the drawings, the sizes of structures may be exaggerated for clarity of illustration. Although the terms "first," "second," etc., may be used herein to describe various elements, these elements should not be limited by these terms. These terms are only used to distinguish one element from another element. For instance, a first element discussed below could be termed a second element without departing from the teachings of the present disclosure. Similarly, the second element could also be termed the first element. As used herein, the singular forms "a," "an," and "the" are intended to include the plural forms as well, unless the context clearly indicates otherwise.

The terms "comprise," "include," "have," etc., when used in this specification, specify the presence of stated features, integers, acts, operations, elements, components, and/or combinations thereof but do not preclude the presence or addition of one or more other features, integers, acts, operations, elements, components, and/or combinations thereof. Further, an expression that an element such as a layer, film, region, substrate or plate is placed "on" or "above" another element indicates not only a case where the element is placed "directly on" or "just above" the other element but also a case where a further element is interposed between the element and the other element. On the contrary, an expression that an element such as a layer, film, region, substrate or plate is placed "beneath" or "below" another element indicates not only a case where the element is placed "directly beneath" or "just below" the other element but also a case where a further element is interposed between the element and the other element.

Unless context clearly indicates otherwise, all numbers, figures and/or expressions that represent ingredients, reaction conditions, polymer compositions, and amounts of mixtures used in the specification are approximations that reflect various uncertainties of measurement occurring inherently in obtaining these figures among other things. For this reason, it should be understood that, in all cases, the term "about" modifies all the numbers, figures and/or expressions. In addition, when numerical ranges are disclosed in the description, these numerical ranges are continuous and include all numbers from the minimum to the maximum including the maximum within the ranges unless otherwise defined. Furthermore, when the range is referred to as an integer, it includes all integers from the minimum to the maximum including the maximum within the range, unless otherwise defined.

A method of producing alkyl acrylate according to the present disclosure may include: synthesizing a catalyst including a zeolite without using an organic structure directing agent; and obtaining alkyl acrylate by contacting the catalyst with alkyl lactate in the presence of alkanol.

One of the characteristic aspects of the method according to the present disclosure may be that the catalyst is synthesized without using an organic structure directing agent.

Specifically, the synthesizing of the catalyst may include: preparing a mixed solution including an aluminum source, a silicon source, an alkali source, and water; obtaining the zeolite by hydrothermal synthesis of the mixed solution; calcining the zeolite; and obtaining the catalyst by adding and reacting the zeolite to a solution including a potassium salt to ion-exchange the zeolite with potassium ions. Here, in the preparing of the mixed solution, the mixed solution may not include an organic structure directing agent.

The zeolite may be represented by the following Chemical Formula 1. However, the zeolite is not limited to the following Chemical Formula 1, and may include any zeolite as long as it belongs to the Mobil-type five (MFI) zeolites in the technical field to which the present disclosure belongs. Specifically, the zeolite may include ZSM-5.

xAl₂O₃·yNa₂O·100SiO₂·2500H₂O [Chemical Formula 1]

In Chemical Formula 1, x may be a number in a range of 1 to 7, and y may be a number in a range of 5 to 15.

As the aluminum source, for example, a water-soluble aluminum-containing compound may be used. Specifically, the water-soluble aluminum-containing compound may be sodium aluminate, aluminum nitrate, aluminum sulfate, aluminum hydroxide, or combinations thereof. In one particular example, the water-soluble aluminum-containing compound is aluminum sulfate. The relative content of the aluminum source and the silicon source may have a silicon (Si)/aluminum (Al) molar ratio in a range of 5:1 to 50:1.

The silicon source may include silica, a silicon-containing compound that can generate silicic acid ions in water, or the like. Specifically, examples thereof include colloidal silica, amorphous silica, tetraethyl orthosilicate, sodium silicate, alumino-silicate gel, or the like. These silicon sources may be used individually or in combination of two or more thereof. In one particular example, the silicon source is sodium silicate.

The alkali source may include sodium hydroxide, potassium hydroxide, rubidium hydroxide, cesium hydroxide, alkaline components of aluminate and silicate, alkali components of alumino-silicate gel, combinations thereof, or the like. The alkali source may be added separately, or may be added in the form of an alkali salt of a silicon source or aluminum source. The content of the alkali source may be such that the alkali metal/aluminum (Al) molar ratio is in a range of 0.8:1 to 1.1:1.

The zeolite may be obtained through hydrothermal synthesis in which the mixed solution is heated to a predetermined temperature in a high-pressure reactor. The pressure and temperature of the reactor are not particularly limited, and may be appropriately adjusted depending on the composition of the desired zeolite and the type of raw material.

The method may further include washing and drying the zeolite obtained as above. The washing is performed for 30 minutes to 180 minutes using at least one selected from deionized water and purified water. In one particular example, the washing is performed using deionized water. The drying may be performed using an oven at a temperature in a range of 90°C to 120°C for 12 to 24 hours.

The calcining of the zeolite may be performed by heating the zeolite at a temperature in a range of 450°C to 600°C for 2 hours to 10 hours. When the temperature is less than 450°C, impurities may remain in the zeolite framework, and when the temperature exceeds 600°C, it may cause strain to the zeolite framework.

Because the zeolite obtained as above is not of the potassium type, the zeolite may be added and reacted to the solution including the potassium salt, and the alkali metal of the zeolite may be ion-exchanged with potassium to obtain the catalyst. Here, when potassium hydroxide is used as the alkali source, the ion exchange act may not be performed.

The ion exchange may be performed by a conventional method. The potassium salt used for the ion exchange may include potassium nitrate (KNO₃), potassium bromide (KBr), potassium chloride (KCl), or combinations thereof. In one particular example, the potassium salt is potassium bromide (KBr).

The catalyst obtained as above may have an Si/Al molar ratio in a range of 5:1 to 50:1 and a potassium (K)/Al molar ratio in a range of 0.6:1 to 1:1. When the molar ratio of each element falls within the above numerical range, the yield of alkyl acrylate can be increased.

In the present disclosure, alkyl lactate reacts in the presence of alkanol. This may be to suppress a reverse reaction in which the produced alkyl acrylate is converted back to alkyl lactate.

The alkanol may be a mono or poly alcohol having 1 to 5 carbon atoms or, in certain examples, a mono alcohol having 1 to 3 carbon atoms.

The alkyl lactate may be obtained as an alkyl ester in purification of lactic acid produced by fermentation, and may be synthesized directly from sugar through a heterogeneous catalyst. In the alkyl lactate, an alkyl group may be a straight-chain or branched hydrocarbon having 1 to 4 carbon atoms. In certain examples, the alkyl group is a straight-chain hydrocarbon having 1 or 2 carbon atoms.

The alkanol and the alkyl lactate may include the same alkyl group.

The obtaining of the alkyl acrylate may be performed by reacting 20% by weight to 90% by weight of the alkanol and 10% by weight to 80% by weight of the alkyl lactate with the catalyst on the basis of the total mass of the alkanol and the alkyl lactate. When the content of the alkyl lactate is less than 10% by weight, a side reaction may occur in olefin synthesis using alkanol as the main reactant, and when the content of the alkyl lactate exceeds 80% by weight, the viscosity of the reactant increases, so it may be difficult to inject the reactant and the reaction flow rate may be non-uniform.

A conversion reaction of the alkyl lactate into the alkyl acrylate may be performed at a temperature in a range of 300°C to 400°C and a pressure in a range of 1 bar to 5 bar.

In addition, the conversion reaction may be caused by gasifying the alkanol and the alkyl lactate and then injecting them into the reactor loaded with a catalyst. Because the alkanol and the alkyl lactate are liquid at room temperature, the alkanol and the alkyl lactate in liquid phase may be mixed in advance and then vaporized, the alkanol and the alkyl lactate may be vaporized by separately injecting them into the reactor, or the alkanol and the alkyl lactate may be vaporized using an inert gas that dilutes them.

The inert gas may include nitrogen, helium, argon, or combinations thereof. In certain examples, the inert gas is nitrogen.

When the gasified reactant is injected into the reactor, the gasified reactant comes into contact with the catalyst provided in the reactor, and a dehydration reaction of the alkyl lactate is promoted on a surface of the catalyst, and an ester in the form of alkyl acrylate, water, and the inert gas, and a small amount of residual reactant remain in the reactor.

The alkyl acrylate may be separated from the gas mixture after the reaction to obtain the alkyl acrylate. As a method of separating the alkyl acrylate, a conventional method may be used, and a boiling point distillation method may be used.

Hereinafter, the present disclosure is described in more detail through specific examples and comparative examples. The following examples and comparative examples are merely illustrative to aid understanding of the present disclosure, and the scope of the present disclosure is not limited thereto.

### <Preparation Example 1> Production of SDA free-K-ZSM-5-10-0.99 (KNO₃) catalyst

A mixed solution was obtained by mixing Colloidal silica (Ludox AS-40, Dupont Chem. Co.,) as a silicon source, about 1.5 g of about 10% by weight NaOH solution prepared from about 96% by weight sodium hydroxide (Samchun Chemical) pellets as an alkali source, about 0.9 g of sodium aluminate (37 wt. % Al₂O₃ and 31 wt. % Na₂O, Junsei Chemical) as an aluminum source, and about 55 g of deionized water prepared using a pure water maker (AQ312C, Youngin Science).

A Teflon container was washed with hydrofluoric acid before each subsequent synthesis to eliminate seed effects caused by residual products that may be generated from previous experiments.

A 10% NaOH solution was added to a about 300 ml Teflon-lined autoclave and washed for about 1 hour under hot water conditions at about 150°C.

The mixed solution was added to the autoclave and the inside of the autoclave was heated to a predetermined temperature to produce zeolite through hydrothermal synthesis.

The zeolite was filtered, washed, dried at about 100°C, and calcined at about 550°C to obtain ZSM-5 produced without using an organic structure directing agent. Here, the Si/Al molar ratio was 10:1.

The ZSM-5 was ion-exchanged with potassium nitrate (KNO₃) to produce a catalyst. Specifically, about 1 g of the ZSM-5 was added to a 1 M KNO₃ aqueous solution and stirred at about 80°C for about 6 to 8 hours to obtain a catalyst (SDA free-K-ZSM-5-10-0.99(KNOs)). In the K-ZSM-5-10-0.99(KNO₃), 10:1 is the Si/Al molar ratio, 0.99:1 is the K/Al molar ratio, and KNO₃ represents the type of potassium salt used during ion exchange with potassium.

### <Preparation Example 2> Production of SDA free-K-ZSM-5-16-1.00 (KNO₃) catalyst

A catalyst (SDA free-K-ZSM-5-16-0.99(KNO₃)) was produced in the same manner as in Preparation Example 1, except that the Si/Al molar ratio was adjusted to 16:1 by using about 0.6 g of sodium aluminate (37 wt.% Al₂O₃ and 31 wt.% Na₂O, Junsei Chemical) as an aluminum source, and the K/Al molar ratio was 1:1.

### <Preparation Example 3> Production of SDA free-K-ZSM-5-22-1.00 (KNO₃) catalyst

A catalyst (SDA free-K-ZSM-5-22-1.00 (KNO₃)) was produced in the same manner as in Preparation Example 2, except that the Si/Al molar ratio was adjusted to 22:1 by using about 0.3 g of sodium aluminate (37 wt.% Al₂O₃ and 31 wt.% Na₂O, Junsei Chemical) as an aluminum source.

### <Comparative Preparation Example 1> Production of Zeolyst-K-ZSM-5-12-0.99 (KNO₃) catalyst

Zeolyst-K-ZSM-5-12-0.99 (KNO₃) was produced by ion-exchanging commercial ZSM-5 (CBV2314, Zeolyst) with an Si/Al molar ratio of 12:1 with KNO₃.

### <Comparative Preparation Example 2> Production of Zeolyst-K-ZSM-5-15-0.80 (KNO₃) catalyst

Zeolyst-KZSM-5-15-0.80 (KNO₃) was produced in the same manner as in Comparative Preparation Example 1, except that commercial ZSM-5 (CBV3024E, Zeolyst) with an Si/Al molar ratio of 15:1 was used.

### <Comparative Preparation Example 3> Production of Zeolyst-K-ZSM-5-25-1.00 (KNO₃) catalyst

Zeolyst-K-ZSM-5-25-1.00 (KNO₃) was produced in the same manner as in Comparative Preparation Example 1, except that commercial ZSM-5 (CBV5524G, Zeolyst) with an Si/Al molar ratio of 25:1 was used.

### <Preparation Example 4> Production of SDA free-K-ZSM-5-10-0.99 (KBr) catalyst

SDA free-K-ZSM-5-10-0.99 (KBr) was produced in the same manner as in Preparation Example 1, except that a 1 M KBr aqueous solution was used instead of a 1 M KNO₃ aqueous solution.

### <Preparation Example 5> Production of SDA free-K-ZSM-5-16-1.00 (KBr) catalyst

SDA free-K-ZSM-5-16-1.00 (KBr) was produced in the same manner as in Preparation Example 2, except that a 1 M KBr aqueous solution was used instead of 1 M KNO₃ aqueous solution.

### <Preparation Example 6> Production of SDA free-K-ZSM-5-22-1.00 (KBr) catalyst

SDA free-K-ZSM-5-22-1.00 (KBr) was produced in the same manner as in Preparation Example 3, except that a 1 M KBr aqueous solution was used instead of a 1 M KNO₃ aqueous solution.

### <Comparative Preparation Example 4> Production of Zeolyst-K-ZSM-5-12-0.99 (KBr) catalyst

Zeolyst-K-ZSM-5-12-0.99 (KBr) was produced in the same manner as in Comparative Preparation Example 1, except that a 1 M KBr aqueous solution was used instead of a 1 M KNO₃ aqueous solution.

### <Comparative Preparation Example 5> Production of Zeolyst-K-ZSM-5-15-0.99 (KBr) catalyst

Zeolyst-K-ZSM-5-15-0.99 (KBr) was produced in the same manner as in Comparative Preparation Example 2, except that a 1 M KBr aqueous solution was used instead of a 1 M KNO₃ aqueous solution.

### <Comparative Preparation Example 6> Production of Zeolyst-K-ZSM-5-25-0.99 (KBr) catalyst

Zeolyst-K-ZSM-5-25-0.99 (KBr) was produced in the same manner as in Comparative Preparation Example 3, except that a 1 M KBr aqueous solution was used instead of a 1 M KNO₃ aqueous solution.

### [Experimental Example 1]

About 2 cc of each catalyst produced in Preparation Examples 1 to 3 and Comparative Preparation Examples 1 to 3 was loaded into a reactor. About 12.5 mol% methyl lactate was vaporized with about 65 mol% methanol and then injected into the reactor.

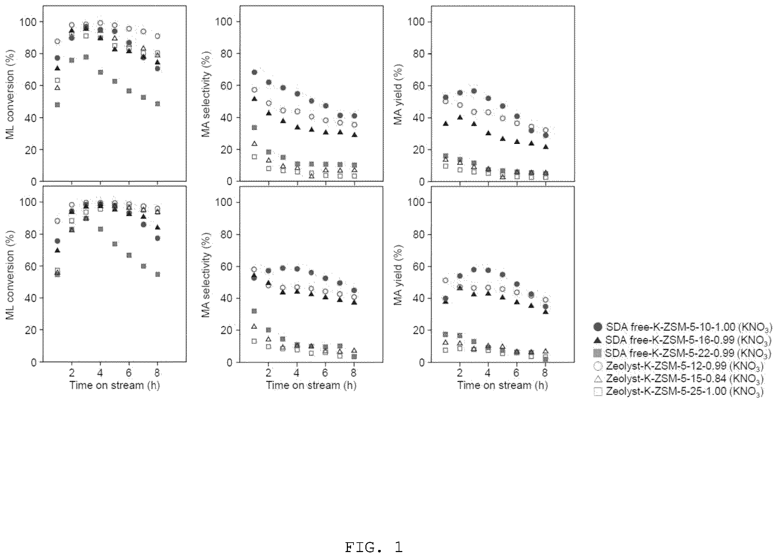

After the reaction proceeded for 8 hours, the concentrations of methyl lactate and methyl acrylate were measured using a method of collecting liquid products captured in a cold trap every hour. The conversion rate of methyl lactate, selectivity to methyl acrylate, and yield of methyl acrylate were measured according to the following Calculation Formulas 1 to 3, and the results are shown in FIG. 1. In FIG. 1, the upper graphs show the results of the reaction at about 340°C, and the lower graphs show the results of the reaction at about 360°C. Conversion rate of methyl lactate (ML conversion, %) = (supplied methyl lactate concentration - unreacted methyl lactate concentration) / supplied methyl lactate concentration × 100 Selectivity of methyl acrylate (MA selectivity, %) = methyl acrylate concentration / resulting product concentration after reaction × 100 Yield of methyl acrylate (MA yield, %) = methyl acrylate concentration / supplied methyl lactate concentration × 100

Referring to FIG. 1, it was confirmed that SDA free-K-ZSM-5(KNOs) produced according to the present disclosure exhibited superior MA selectivity and yield during the reaction time for each Si/Al molar ratio compared to Zeolyst-K-ZSM-5(KNO₃) in a methyl lactate conversion reaction. These results indicate that SDA free-K-ZSM-5 is excellent in terms of methyl acrylate production yield through a dehydration process of methyl lactate and is a very useful catalyst in the process.

### [Experimental Example 2]

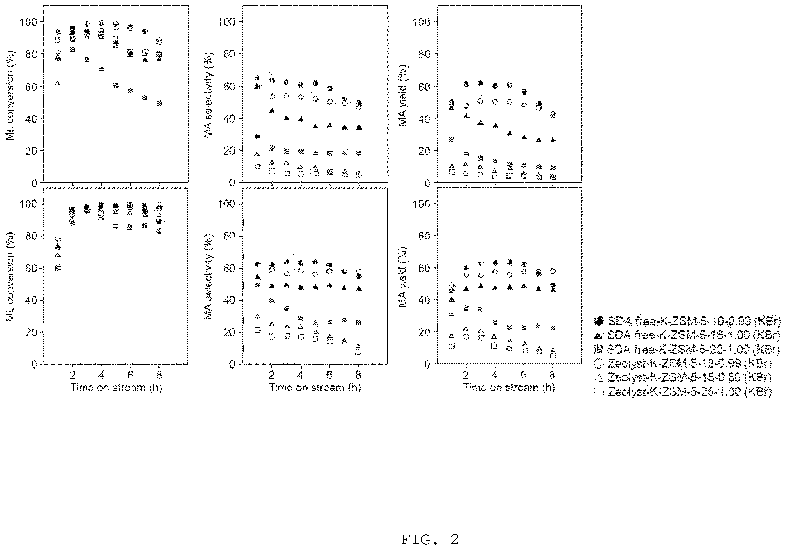

An experiment was performed in the same manner as in Experimental Example 1 using each catalyst produced in Preparation Examples 4 to 6 and Comparative Preparation Examples 4 to 6. The measurement results are shown in FIG. 2.

Referring to FIG. 2, it was confirmed that SDA free-K-ZSM-5(KBr) produced according to the present disclosure exhibited superior MA selectivity and yield during the reaction time compared to Zeolyst-K-ZSM-5(KBr) in a methyl lactate conversion reaction. In addition, in FIG. 2, it was confirmed that compared to the KNO₃ ion exchange catalyst (FIG. 1), the MA yield was excellent as the reaction time elapsed. These results indicate that SDA free-K-ZSM-5(KBr) is the best in terms of methyl acrylate production yield through a dehydration process of methyl lactate and has long-term operability.

### [Experimental Example 3]

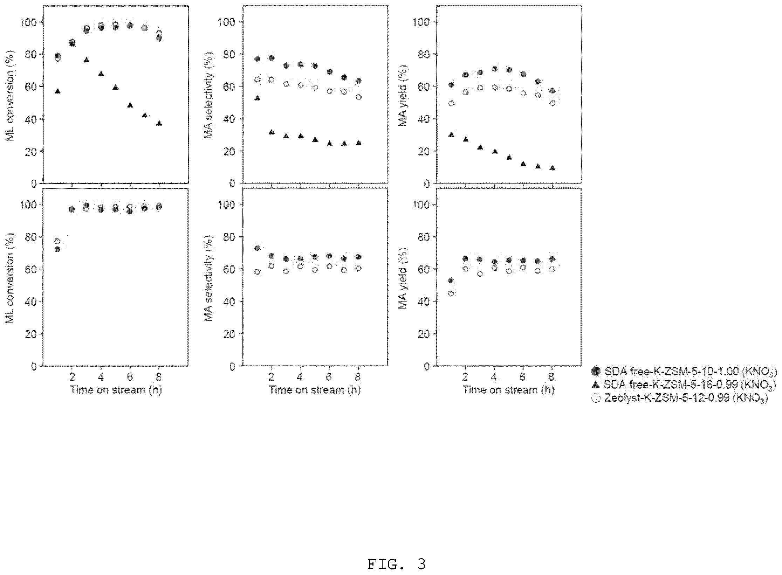

An experiment was performed in the same manner as in Experimental Example 1 except that the concentration of methyl lactate among the reactants was set to about 5.6 mol%. The measurement results are shown in FIG. 3.

Referring to FIG. 3, it was confirmed that compared to Experimental Example 1, higher MA selectivity and yield were achieved when about 5.6 mol% of methyl lactate was used.

These results indicate that the process conditions can be used as conditions to further increase production yield.

### [Experimental Example 4]

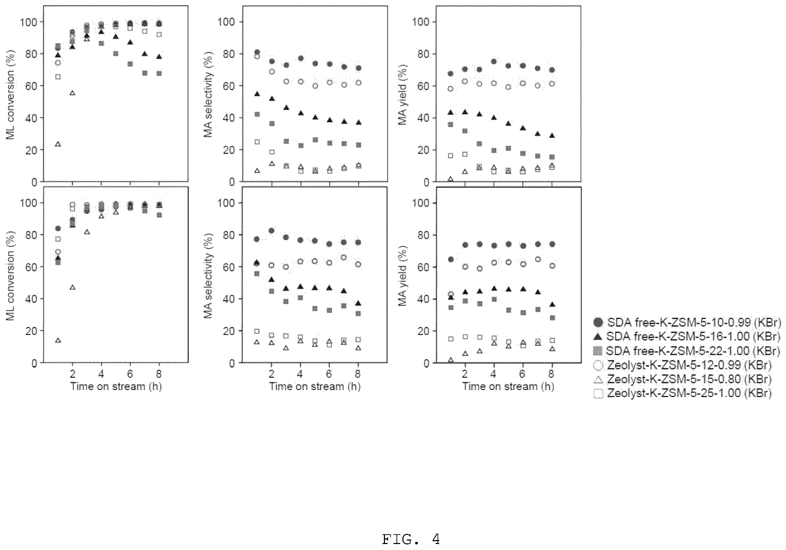

An experiment was performed in the same manner as in Experimental Example 2 except that the concentration of methyl lactate was set to about 5.6 mol%. The measurement results are shown in FIG. 4.

Referring to FIG. 4, it was confirmed that compared to Experimental Example 2, higher MA selectivity and yield were achieved when about 5.6 mol% of methyl lactate was used.

Even when compared to Experimental Examples 1 to 3, these results show the highest MA selectivity and yield, indicating that the process conditions and catalyst utilization can be used as conditions that can best increase production yield.

Although the embodiments of the present disclosure have been disclosed for illustrative purposes, those skilled in the art will appreciate that various modifications, additions, and substitutions are possible, without departing from the scope and spirit of the present disclosure as disclosed in the accompanying claims.

## Claims

1. A method of producing alkyl acrylate, the method comprising:
synthesizing a catalyst comprising zeolite without using an organic structure directing agent; and
obtaining the alkyl acrylate by contacting alkyl lactate with the catalyst in presence of an alkanol.

2. The method of claim 1, wherein the synthesizing of the catalyst comprises:
preparing a mixed solution comprising an aluminum source, a silicon source, an alkali source, and water;
obtaining the zeolite by hydrothermal synthesis of the mixed solution;
calcining the zeolite; and
obtaining the catalyst by adding and reacting the zeolite with a solution comprising a potassium salt to ion-exchange the zeolite with potassium ions,
wherein, in the preparing of the mixed solution, the mixed solution does not comprise the organic structure directing agent.

3. The method of claim 1, wherein the zeolite is represented by Chemical Formula 1:
xAl₂O₃·yNa₂O·100SiO₂·2500H₂O [Chemical Formula 1]
wherein:
x is a number in a range of 1 to 7; and
y is a number in a range of 5 to 15.

4. The method of claim 2, wherein the potassium salt comprises potassium bromide (KBr).

5. The method of claim 1, wherein the catalyst has an Si/Al molar ratio in a range of 5:1 to 50:1.

6. The method of claim 1, wherein the catalyst has a K/Al molar ratio of 0.6:1 to 1:1.

7. The method of claim 1, wherein the alkanol and the alkyl lactate comprise a same alkyl group.

8. The method of claim 1, wherein the obtaining of the alkyl acrylate is performed at temperature in a range of 300°C to 400°C.

9. The method of claim 1, wherein the obtaining of the alkyl acrylate is performed by reacting 20% by weight to 90% by weight of the alkanol and 10% by weight to 80% by weight of the alkyl lactate with the catalyst.

10. A method of producing a catalyst that promotes a reaction for producing alkyl acrylate from alkyl lactate, the method comprising:
preparing a mixed solution comprising an aluminum source, a silicon source, an alkali source, and water;
obtaining zeolite by hydrothermal synthesis of the mixed solution;
calcining the zeolite; and
obtaining the catalyst by adding and reacting the zeolite with a solution comprising a potassium salt to ion-exchange the zeolite with potassium ions,
wherein, in the preparing of the mixed solution, the mixed solution does not comprise an organic structure directing agent.

11. The method of claim 10, wherein the zeolite comprises ZSM-5.

12. The method of claim 10, wherein the zeolite is represented by Chemical Formula 1:
xAl₂O₃ .yNa₂O·100SiO₂ ·2500H₂O [Chemical Formula 1]
wherein:
x is a number in a range of 1 to 7; and
y is a number in a range of 5 to 15.

13. The method of claim 10, wherein the potassium salt comprises potassium bromide (KBr).

14. The method of claim 10, wherein the catalyst has an Si/Al molar ratio in a range of 5:1 to 50:1.

15. The method of claim 10, wherein the catalyst has a K/Al molar ratio in a range of 0.6:1 to 1:1.
